# EUROPEAN PATENT APPLICATION

(11) **EP 2 708 209 A1**
(43) Date of publication of application: **19.03.2014**
(21) Application number: 12382348.6
(22) Date of filing: 13.09.2012
(51) Int. Cl.: A61F 2/14

(54) **Scleral epimacular implant**

(71) Applicant: AJL Ophthalmic, S.A., 01510 Miñano (Alava) (ES); Nadal Reus, Jerónimo, 08017 Barcelona (ES)
(72) Inventor: Nadal Reus, Jerónimo, 08017 Barcelona (ES); Salazar Salegui, Pedro José, 01510 Miñano (Álava) (ES); Pulido, José, 01510 Miñano (Álava) (ES); Barraquer Compte, Rafael Ignacio, 01510 Miñano (Álava) (ES)
(74) Representative: Stiebe, Lars Magnus

(57) **Abstract**

The scleral epimacular implant comprises an arm (6) with a curved configuration, having a first end (1), which can be affixed to a retina (8). The arm (6) comprises a second end (3) comprising a discoidal element (4), whose internal face facing the curvature of the arm (6) is constituted as an indentation platform (5), configured to be in contact with the retina (8) when the implant is placed in said retina (8).

## Description

### TECHNICAL FIELD OF THE INVENTION

The present invention relates to an epimacular implant applicable in the field of medicine, and more specifically, in the field of ophthalmology for the treatment of patients affected by retinal detachment or patients with high degrees of myopia affected by the splitting of the retina by means of vitrectomy surgery.

### BACKGROUND OF THE INVENTION

Nowadays, a high percentage of patients affected by retinal detachment due to a macular hole are cured with a classic surgery through vitrectomy and the additional use of intraocular internal tamponades, such as expansive gases like SF6 or C3F8, and even with intraocular silicone oil. The retina is a fine layer covering the internal wall of the eyeball. However, in certain cases, when the size of the anteroposterior axis is too long, for example, longer than 30 mm, taking 24 mm as the standard or normal value, the surgeries aimed at obtaining an internal relaxation of the eye are not able to put the retina into contact with the pigment epithelium and the sclera or sclerotic, so that the retinal detachment persists. In these cases, the retina can be readapted by bringing the retina closer to the sclera by means of a technique known as posterior indentation, in order to obtain a retinal adaptation; this effect is carried out by means of the external arrangement of an epimacular implant at a scleral level.

An example of an implant as the one previously mentioned is found in IMO magazine. Said implant is made from silicone and is configured as a curved strap, in the form of a belt, in order to encircle the eyeball and reapply the detached retina or the slit retina in patients with high degrees of myopia. This technique, also known as macular indentation, is associated to vitrectomy surgery, in the cases of retinal detachment due to a macular hole, that is to say, due to the lack of tissue in the central part of the retina; or retinoschisis, that is to say, the gradual splitting of the layers of the retina in patients with high degrees of myopia. Therefore, it could be said that the macular indentation consists in the placement of said implant.

More specifically, the implant described in the previous paragraph consists of a silicone piece with titanium threads within. The piece is placed in the lateral and posterior part of the eyeball to modify its curvature and keep it stable in a corrected position, so that the retina detached due to the elongation of the eyeball is once again placed in its correct position.

This technique is applied to people with retina problems caused by high degrees of myopia, in which the eyeball is more elongated than normal and produces an excessive stretching of the retina, causing its detachment or its splitting into layers, directly resulting in vision problems for the patient. The placement of the silicone piece presses the posterior part of the eyeball, decreasing the excessive elongation of its wall. This way, the piece acts as a belt that is able to return the eyeball to a more appropriate length so that the retina remains applied, that is to say, in contact with the internal wall of the eyeball, thus preventing the production of further detachments or the degeneration of the layers of the retina due to separation, which can be recurrent in abnormally elongated eyes. In many cases, the macular indentation replaces the silicone oil treatment, a fluid that is introduced into the eyeball after some vitrectomies in order to keep the retina adhered to the wall of the eyeball. The results are much better with the macular indentation because it is a definitive technique that prevents the production of long-term problems associated to silicone oil in the majority of the cases, such as glaucoma or the progressive atrophy of the optic nerve.

However, current implants, such as the one previously described, are not exempt of disadvantages, which is in detriment of macular indentation as the optimal solution. In that sense, it should be mentioned that, given its configuration, its application in the posterior part of the eyeball does not produce a sufficient correcting effect in many cases. In addition, its placement is extremely difficult, especially in respect of finding its precise optimal position to achieve the desired correction. On the other hand, with the purpose of achieving the desired corrective effects, these implants must incorporate sufficiently rigid elements, which is achieved by means of the incorporation of threads and other metallic components. However, the metallic elements are not biocompatible, which means, according to different national legislations, that they might not be usable in certain countries, such as, for example, the United States.

All of these disadvantages have made this implants not to be widely used nowadays, which, in turn, means that their development is not pursued, also, in part because the patient population they could be aimed at is not very broad.

### DESCRIPTION OF THE INVENTION

The present invention relates to a scleral epimacular implant, intended to be implanted in the eye of patients with severe retinal detachment.

The implant comprises an arm with a curved configuration, in which the curvature of the arm is obviously selected so that it offers an optimal correction according to the curvature of the retina to be corrected.

Said arm has a first end which can be affixed to a retina by its posterior part, like in the macular indentation techniques exposed in the previous section.

Therefore, according to the invention, the arm comprises a second end comprising a discoidal element, whose internal face facing the curvature of the arm is constituted as an indentation platform configured to be in contact with the retina when the implant is placed in said retina. This way, due to its placement and affixing to the eye through the area corresponding to the first end, when the implant is placed in the eye, the indentation platform presses the retina, thus achieving the correction in its curvature.

With the purpose of facilitating its placement and boosting its leverage effect in its affixing to the eye, the invention contemplates that the first ends comprise a pair of protrusions.

Therefore, it is basically a spoon-shaped device comprising two ends. The affixing is carried out through the first end, normally by means of sewing to the eye, while the second end presents the discoidal element, which is left pressed against the retina, holding it and thus solving the pathology.

The geometrical or structural shape of the implant is a very important aspect in order to facilitate the placement of the device in the patient. Two extremely important aspects can be highlighted in this sense.

On the one hand, the arm presents a continuous curvature angle, which is always the same and matches the angle of the eyeball. The arm is longer than the one known until now, published by IMO magazine, which allows it to be used in larger eyes, thus facilitating its placement. Likewise, the invention contemplates that the arm has a configuration of a flat curved element, and may present, according to a specific embodiment, two affixing points to the sclera or sclerotic, the most external layer of the eyeball and where the device is "sown": one of this points is the end of the arm that is furthest away from the platform and the other through an intermediate part. In the case that sufficient stability in the placement is not achieved only with the affixing of the first end, said stability is achieved with the other additional points. Usually, said sutures are made with nylon sutures.

On the other hand, the invention contemplates the possibility that the transition between the arm and the discoidal element in the second end is made by means of a step, so that the indentation platform is located closer towards the interior of the curvature of the arm than the internal face of the arm itself. The pressure effect that the indentation platform must carry out is reinforced with this characteristic.

Likewise, a preferred embodiment of the invention contemplates a curved-convex indentation platform. According to said embodiment, the platform does not present a flat upper part, which is likewise contemplated as a variant of embodiment, but a convex surface, which allows an excellent integration and a better adjustment to the posterior part of the eyeball, which also presents a similar shape. This shape allows the placement of the invention in positions with a certain margin, which was not possible in the flat surface, which requires its placement in an exact and precise point, obviously making said placement more difficult.

In the case of the implant described in IMO magazine, the part of the device applied to the posterior part of the eyeball is flat and nail-shaped, which makes its placement in the appropriate position very difficult since it must be understood that this posterior part presents a spherical shape, which is why it is not easy to find the exact placement position.

On the other hand, the invention contemplates that the implant of the invention comprises PMMA, polymethylmethacrylate. In the case of the implant of the IMO magazine, it is metallic regarding the nerves it incorporates to provide sufficient rigidity to the implant, due to which it is not compatible in addition to not presenting the discoidal end of the invention. Likewise, it is contemplated that the implant comprises an external coating of medical grade silicone.

### DESCRIPTION OF THE DRAWINGS

Next, in order to supplement the description being made and with the purpose of helping to provide a better comprehension of the characteristics of the invention, a set of drawings according to a preferred practical embodiment thereof, where the following has been represented by way of illustration and not by way of limitation, is attached as an integral part of said description:

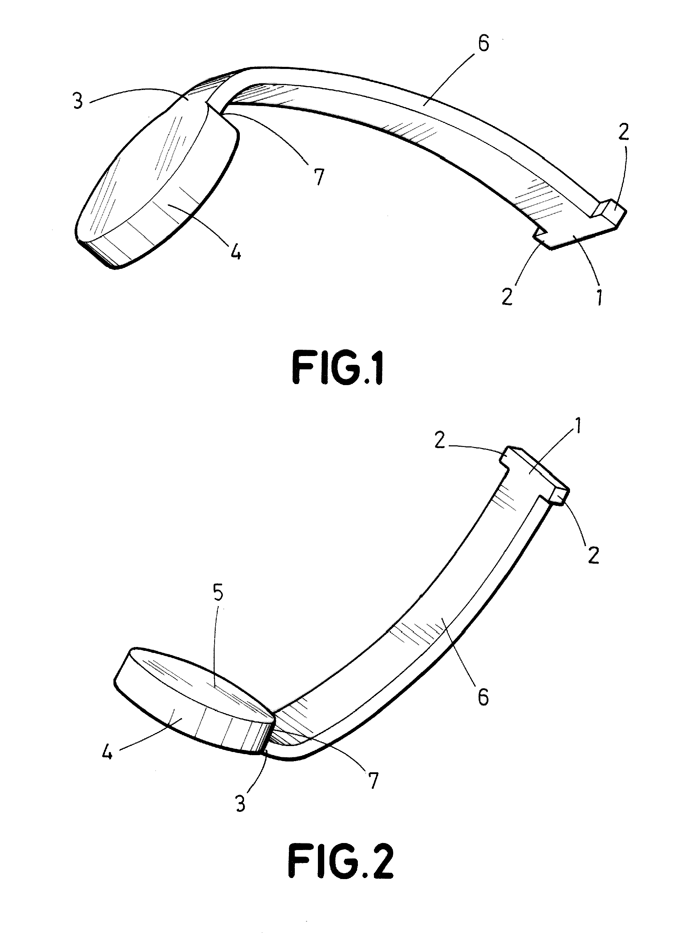

Figure 1.- Shows a schematic perspective view of an embodiment of the implant of the invention.
Figure 2.- Shows another perspective view of the implant represented in Figure 1.
Figure 3.- Shows another perspective view of the implant represented in Figures 1 and 2.
Figure 4.- Shows a detail in which the arrangement of the implant in the eye of a patient can be observed.
Figure 5.- Shows a schematic detail of an embodiment of the discoidal element, according to an elevational view..
Figure 6.- Shows a detail of a variant of the embodiment of the discoidal element.
Figure 7.- Shows a preferred embodiment of the implant of the invention as the one represented schematically in the detail of Figure 5.

### PREFERRED EMBODIMENT OF THE INVENTION

Based on the described figures, we can observe how in one of the possible embodiments of the invention, the implant of the invention proposes to be comprised by one arm (6) with a curved configuration having a first end (1), which can be affixed to a retina (8), and a second end (3) comprising a discoidal element (4), whose internal face facing the curvature of the arm (6) is constituted as an indentation platform (5), configured to be in contact with the retina (6) when the implant is placed in said retina (8). In order to facilitate its placement, the first end (1) comprises a pair of protrusions (2).

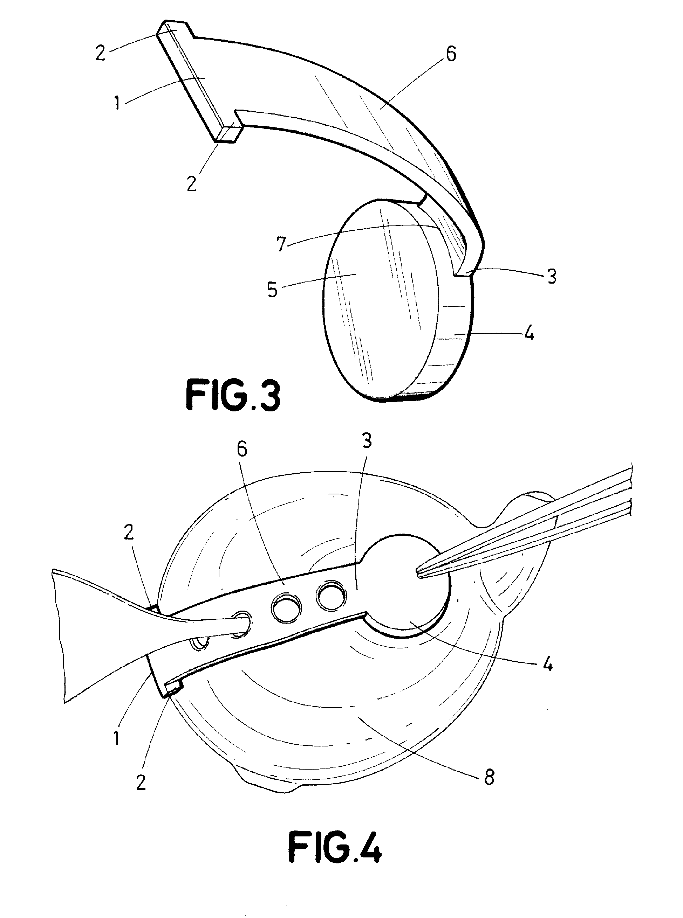

The arm (6) presents a continuous curvature angle, which is always the same and matches the angle of the eyeball. Likewise, the arm (6) has the configuration of a flat curved element, being able to present, as observed in the embodiment of figure 4, two affixing points to the sclera. One of these points is the end of the arm that is furthest away from the platform, and the other through an intermediate part.

The transition between the arm (6) and the discoidal element (4) in the second end (3) is carried out by means of a step (7), so that the indentation platform (5) is located closer towards the interior of the curvature of the arm (6) than the internal face of the arm (6) itself.

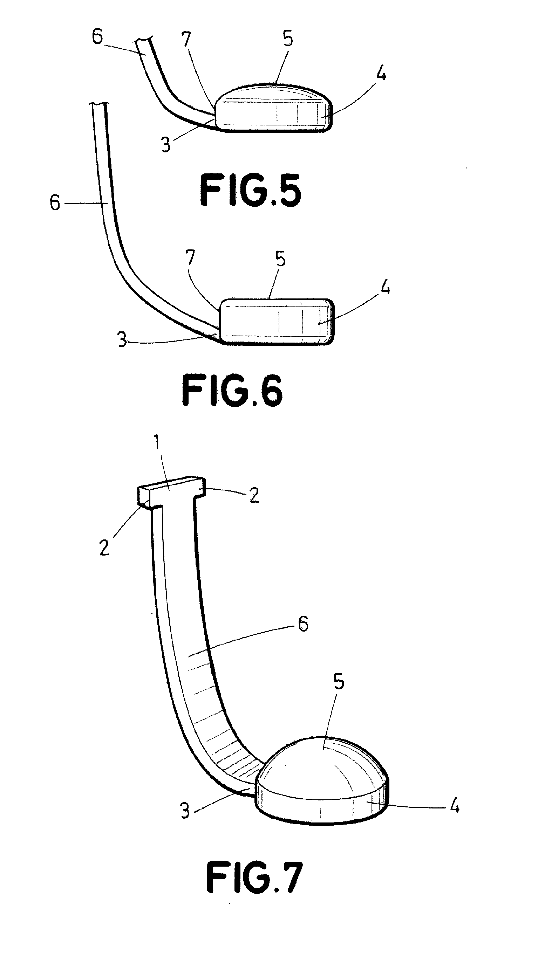

According to a preferred embodiment, as represented in figures 5 and 7, the indentation platform (5) is curved-convex. According to said embodiment, the platform does not present a flat upper part, as represented in the remaining figures, but a convex surface, which allows an excellent integration and a better adjustment to he posterior part of eyeball, which presents a similar shape. This shape allows it to be placed in positions with a certain margin, which was not possible with the flat surface, requiring its placement at an exact and precise point, which obviously makes said placement more difficult. The indentation platform (5) is wider than the one known by IMO magazine, about 8 mm, and can be 3-3-2 mm high from its base in the highest part of the convexity, in order to generate a better indentation over the retina. The arm (6) can be between 3 to 4 mm wide, so it occupies less space.

On the other hand, a preferred embodiment of the implant of the invention is exclusively manufactured in PMMA, polymethylmethacrylate, with an external silicone coating. Therefore, the body is manufactured from PMMA with an external scleral curvature radius analogue to the physiological one in patients with a large anteroposterior axis, coated with a hard silicone layer with a flat epimacular indentation platform with a radius of 8 mm, achieving an approximation of the sclera towards the retina due to the effect of flattening over the same. Unlike other older silicone implants with metallic elements inside, the implant of the invention uses PMMA as a structural element providing it with the necessary rigidity. Both materials, PMMA and silicone, are biocompatible and their tolerance is well known. In addition, this provides the set with the necessary rigidity, since this implant cannot present any flexibility because it would not act correctly; its function is to "push" or press the eyeball, provoking a correct location of the retina displaced due to the elongation of the eyeball, that is to say, it exerts pressure on it through its back part, and said part must be kept in this manner indefinitely.

Based on this description and the set of figures, the expert in the art will be able to understand that the embodiments of the invention described can be combined in multiple manners within the object of the invention. The invention has been described according to certain preferred embodiments thereof, but it will be evident for the expert in the art that multiple variations can be introduced in said preferred embodiments without exceeding the object of the claimed invention.

## Claims

1. Scleral epimacular implant comprising an arm (6) with a curved configuration having a first end (1), which can be affixed to a retina (8), **characterized in that** the arm (6) comprises a second end (3) comprising a discoidal element (4), whose internal face facing the curvature of the arm (6) is constituted as an indentation platform (5), configured to be in contact with the retina (8) when the implant is placed in said retina (8).

2. Implant according to claim 1, in which the first end (1) comprises a pair of protrusions (2).

3. Implant according to any one of the previous claims, in which the arm (6) has a configuration of a flat, curved element.

4. Implant according to any one of the previous claims, in which the transition between the arm (6) and the discoidal element (4) in the second end (3) is carried out by means of a step (7), so that the indentation platform (5) is located closer towards the interior of the curvature of the arm (6) than the internal face of the arm (6) itself.

5. Implant according to any one of the previous claims, in which the indentation platform (5) is curved-convex.

6. Implant according to any one of the previous claims, comprising PMMA.

7. Implant according to any one of the previous claims, comprising an external silicone coating.

8. Implant according to any one of the previous claims, exclusively manufactured in PMMA with an external silicone coating.
